# EUROPEAN PATENT SPECIFICATION

(11) **EP 4 043 511 B1**
(45) Date of publication and mention of the grant of the patent: **09.09.2026**
(21) Application number: 22167310.6
(22) Date of filing: 14.03.2017
(51) Int. Cl.: C08G 63/16, C07D 307/08, C08G 63/78

(54) **COMBINED PROCESS FOR THE PRODUCTION OF TETRAHYDROFURAN AND POLYESTERS COMPRISING 1,4-BUTYLENE DICARBOXYLATE UNITS**
KOMBINIERTES VERFAHREN ZUR HERSTELLUNG VON TETRAHYDROFURAN UND POLYESTERN MIT 1,4-BUTYLENDICARBOXYLATEINHEITEN
PROCÉDÉ COMBINÉ DE PRODUCTION DE TÉTRAHYDROFURANE ET DE POLYESTERS COMPRENANT DES UNITÉS DICARBOXYLATE DE 1,4-BUTYLÈNE

(30) Priority: 14.03.2016 IT UA20161612
(43) Date of publication of application: 17.08.2022
(60) Divisional application: 26196471.2
(62) Document number(s) of the earlier application(s) in accordance with Art. 76 EPC: 17714401.1 / 3 430 070
(73) Proprietor: Novamont S.p.A., 28100 Novara (IT)
(72) Inventor: MILIZIA, Tiziana, 28100 Novara (IT); MAZZA, Emilio, 00199 Roma (IT)
(74) Representative: Zanoli, Enrico

(56) References cited:
- DE-A1- 10 021 703
- US-A- 4 680 376
- US-A1- 2008 161 530
- US-B1- 6 232 435

## Description

This invention relates to a combined process for the production of tetrahydrofuran and polyesters and copolyesters comprising repetitive 1,4-butylene dicarboxylate units.

Polyesters comprising repetitive 1,4-butylene dicarboxylate units, such as for example poly(1,4-butylene terephthalate), poly(1,4-butylene succinate), poly(1,4-butylene sebacate), poly(1,4-butylene terephthalate-co-1,4-butylene adipate) are widely used at the present time because of their excellent mechanical and workability properties in all fields in which thermoplastic polymer materials are used, such as fibres, moulded articles and blow-moulded articles, including films.

It is known that during the synthesis of polyesters of this type, which comprises the condensation reaction of 1,4-butanediol with diacids or their derivatives, depending upon the reaction conditions tetrahydrofuran (THF) can form, mainly deriving from the 1,4-butanediol dehydration reaction during synthesis of the polyester:

HO(CH₂)₄OH → THF + H₂O

and from degradation of the terminal hydroxybutyl groups which form during synthesis of the polyester itself ("backbiting" reaction):

polyester-C(O)O(CH₂)₄OH → THF+ polyester-C(O)OH

The generation of THF generally results in undesired consumption of 1,4-butanediol with consequent particular disadvantages from the point of view of productivity and the competitiveness of processes for synthesis of the said polyesters. Furthermore, it leads to the need to develop suitable treatments to remove and dispose of the same, together with other process by-products. In general, the THF formed is removed together with the other volatile components and by-products produced during the normal synthesis processes for the said polyesters, mainly during the esterification/transesterification steps in the said processes, making its recovery difficult and inconvenient.

US 4,680,376 concerns a process for continuous production of polybutyleneterephthalate by direct esterification of terephthalic acid and 1,4-butanediol in the presence of Sn or Ti containing catalysts with minimal formation of tetrahydrofuran.

US 2008/161530 A1 discloses a process for the synthesis of elastomeric copolyesters from polybutyleneterephthalate and polyoxytetramethylene glycol in which the tetrahydrofuran obtained as a byproduct in the synthesis of polybutyleneterephthalate from 1,4-butanediol is rectified and then reacted to form polyoxytetramethylene glycol.

US 6,232,435 B1 discloses a process for preparing polyalkylene arylates by esterifying or transesterifying an aromatic bicarboxylic acid with a molar excess of an aliphatic dihydroxycompound and polycondensing the resultant esterification or transesterification product.Starting from this technical background the present invention relates to a process capable of combining both the competitiveness and productivity requirements associated with the production of polyesters comprising 1,4-butylene dicarboxylate units and the possibility of efficiently recovering the tetrahydrofuran formed during synthesis of the said polyesters, with a high degree of purity.

In particular, this invention relates to a combined process for the production of tetrahydrofuran and polyesters comprising 1,4-butylene dicarboxylate units, comprising the steps of:
1. subjecting a mixture comprising 1,4-butanediol and at least one dicarboxylic acid, an ester, a salts or a derivative thereof to esterification and/or transesterification to produce an oligomer and a vapour phase comprising tetrahydrofuran and 1,4-butanediol;
2. separating the vapour phase comprising tetrahydrofuran and 1,4-butanediol from the oligomer;
3. distilling the vapour phase in a distillation column comprising at least one plate section and at least one packed section;
4. separating a light fraction comprising tetrahydrofuran and less than 1% by weight, preferably less than 0.5% by weight, of 1,4-butanediol from a heavy fraction comprising 1,4-butanediol;
5. polycondensing the oligomer obtained from step 1; wherein at least some of the components of the mixture of step 1 are premixed, before being delivered to the reactor.

Step 1 of the process of the invention provides for an esterification and/or transesterification reaction of a starting mixture comprising at least one dicarboxylic acid and/or an ester, a salt or a derivative thereof with a diol component comprising 1,4-butanediol or a mixture thereof with at least one diol different from 1,4-butanediol. The esterification and/or transesterification reaction leads to the formation of an oligomer and a vapour phase comprising unreacted 1,4-butanediol, possibly in mixture with the other diols added to the starting mixture, tetrahydrofuran resulting from the dehydration of 1,4-butanediol, water (if the starting mixture includes dicarboxylic acids), and/or alcohols (if the starting mixture comprises dicarboxylic acid esters).

At least some of the components of the starting mixture of step 1 are premixed, preferably at T<70°C, before being delivered to the reactor. It is also possible to premix some of the components and subsequently add the remaining ones, for example during the esterification/transesterification reaction.

In the case of polyesters in which the dicarboxylic component comprises repetitive units deriving from several dicarboxylic acids, whether aliphatic or aromatic, it is also possible to premix some of these with the aliphatic diols, preferably at T < 70 °C, adding the remaining part of the dicarboxylic acids, diols and any other co-monomers to the esterification/transesterification reactor.

In the starting mixture for the process according to this invention the molar ratio between the diols and the dicarboxylic acids, their esters or their salts (the MGR ratio) is advantageously between 1 and 2.5, preferably between 1.5 and 2.1.

Step 1 of the process according to this invention is preferably carried out in the presence of an esterification/transesterification catalyst.

The esterification/transesterification catalyst, which may also advantageously be used as a component of the catalyst for the polycondensation step 5, may in turn be fed directly to step 1 of the process according to this invention and may also first be dispersed in an aliquot of one or more dicarboxylic acids, their esters or salts, and/or of diols to promote a homogenous dispersion in the reaction medium. In a preferred embodiment, the esterification/transesterification catalyst is an organometallic compound comprising tin (for example stannoic acid derivatives), titanium (for example titanates such as tetrabutyl orthotitanate or tetra(isopropyl)orthotitanate), zirconium (for example zirconates such as tetrabutyl orthozirconate or tetra(isopropyl)orthozirconate), antimony, cobalt, lead, aluminium (for example Al-triisopropylate) zinc, and mixtures thereof.

As far as the organometallic esterification/transesterification catalysts of the type mentioned above are concerned, in step 1 of the process according to this invention they are preferably used in concentrations between 8-120 ppm of metal with respect to the quantity of polyester which can theoretically be obtained by converting all the dicarboxylic acids fed to the reactor. In a preferred embodiment, the catalyst used in the esterification/transesterification step is a titanate, more preferably diisopropyl triethanolamine titanate, preferably used at a concentration of 8-120 ppm of metal with respect to the quantity of polyester which can theoretically be obtained by converting all the dicarboxylic acid fed to the reactor.

Step 1 of the process according to the invention is preferably carried out at a temperature of 190-250°C, at a pressure of 0.7-1.5 bar, and over 4 to 10 hours, preferably 5-7 hours. Said reaction conditions make it possible to obtain a vapour phase which is particularly suitable for the efficient recovery of THF with a high degree of purity in step 4 of the process, thus combining the competitiveness and productivity requirements associated with the production of polyesters containing 1,4-butylene dicarboxylate units and the possibility of effectively recovering the tetrahydrofuran formed during synthesis of the said polyesters.

Step 1 of the process according to this invention may be carried out in a single esterification reactor or in two or more esterification reactors arranged in series. Where not explicitly stated otherwise, process configurations which include two or more esterification reactors placed in series are also meant to be included when reference is made in this invention to the process carried out in an esterification reactor.

It has also surprisingly been found that the use of a configuration which provides for two or more esterification reactors in the process of the invention, makes it possible to obtain improved results in terms of balance between the competitiveness and productivity requirements associated with the production of polyesters comprising 1,4-butylene dicarboxylate units and the possibility of effectively recovering the tetrahydrofuran formed during the process.

In a preferred embodiment, step 1 of the process according to this invention is carried out using two esterification reactors placed in series, in which the first reactor operates at a temperature of 190-230°C and a pressure 0.7-1.5 bar, with a residence time of 3 - 7 hours, and the second reactor operates at a temperature of 205 - 250°C and a pressure of 0.7 -1.5 bar, with a residence time of 1 - 3.5 hours.

At the end of step 1 of the process of the invention an oligomer having Mn < 5000, an inherent viscosity of 0.03 - 0.15 dl/g and an acidity of 400 meq/kg or below is preferably obtained.

The Mn and Mw molecular weights may be measured using Gel Permeation Chromatography (GPC). The determination may be performed with the chromatograph system held at 40°C, using a set of three columns in series (particle diameter 5 µm and porosities of 500 A, 10000 A and 100000 A respectively), a refractive index detector, chloroform as eluent (flow 1 ml/min) and using polystyrene as a reference standard.

The terminal acid groups content may be measured in the following way: 1.5 - 3 g of the sample to be analysed are placed in a 100 ml flask together with 60 ml of chloroform. After the sample has completely dissolved 25 ml of 2-propanol are added and immediately prior to the analysis 1 ml of deionised water. The solution so obtained is titrated against a previously standardised solution of NaOH in ethanol. An appropriate indicator, such as for example a glass electrode for acid-base titrations in non-aqueous solvents, is used to determine the end point of the titration. The terminal acid groups content is calculated on the basis of the consumption of NaOH solution according to the following equation: $Terminal acid groups content \left(meq/kg\right) = \frac{\left⌊\left({V}_{eq}-{V}_{b}\right)⋅T\right⌋ ⋅ 1000}{P}$ in which:
V_{eq} = ml of NaOH solution in ethanol at the end point of the titration of the sample;
V_{b} = ml of NaOH solution in ethanol required to reach a pH = 9.5 during the blank titration;
T = concentration of the NaOH solution in ethanol expressed as moles/litre;
P = weight of the sample in grams.

In a preferred embodiment of the process of the invention, the catalyst used in step 1 and the oligomer resulting from the esterification and/or transesterification reaction are fed to the polycondensation step 5 of the process.

In step 2 of the process the vapour phase comprising tetrahydrofuran and 1,4-butanediol is separated from the oligomer and subsequently distilled (according to step 3 of the process) in at least one distillation column, in order to separate a light fraction comprising tetrahydrofuran and less than 1% by weight, preferably less than 0.5% by weight, of 1,4-butanediol from a heavy fraction comprising 1,4-butanediol.

Step 3 of the process according to this invention is preferably carried out in a single distillation column, but may also be carried out using configurations which provide for two or more distillation columns. Where not explicitly described otherwise, process configurations which include two or more distillation columns are also meant when reference is made in this invention to a process carried out in a distillation column.

The distillation column used in step 3 of the process comprises at least one plate section and at least one packed section. As far as the packed section is concerned, this may include Raschig rings, Berl saddles, Pall rings, Intalox saddles, Lessing rings, or a structured packing such as for example Sulzer Mellapak, Koch-Glitsch Flexipac, Gauze. As far as the plate section is concerned, this may comprise bubble cap trays, valve trays, sieve trays or a mixture thereof. According to a preferred embodiment of this aspect of the invention the distillation column used in step 3 comprises one or more plate sections including only bubble cap trays. In a preferred embodiment, the distillation column used in step 3 of the process of the invention comprises a lower part provided with at least one distillation plate, more preferably provided with 1 to 6 plates, even more preferably provided with 2 to 5 plates, and at least one distillation packing in the upper part, more preferably comprising from 13 to 20 theoretical plates, even more preferably comprising or consisting of structured packing. Working with this configuration the vapour phase separated during step 2 of the process is preferably fed to the distillation column in the plate section. Preferably, the part of the distillation column provided with at least one plate constitutes the 15-35 % of the column, and the part provided with packing constitutes the 85-65 % of the column.

Preferably, the part of the distillation column provided with at least one plate can be advantageously used to avoid dragging phenomena when solid monomers are fed in the esterification/transesterification reactor.

The distillation column preferably operates at pressures between 500 mbar and 1200 mbar with bottom temperatures of between 150 and 195 °C, head temperatures between 80 and 98 °C and using a reflux ratio of preferably between 0.5 and 2.

Depending upon the desired degree of purity and specific applications, the light fraction separated off from the head of the distillation column may be passed to subsequent purification steps, such as for example distillation, adsorption, liquid-liquid extraction, or pervaporation steps or combinations thereof.

The heavy fraction separated at the base of the column in step 4 essentially contains 1,4-butanediol, and may advantageously be recycled to step 1 of the process.

Polycondensation step 5 of the process of the invention is preferably carried out in the presence of a catalyst, which may or may not be the same of that used in step 1. Preferably the catalyst used in polycondensation step 5 of the process is selected from organometallic compounds comprising tin (for example stannoic acid derivatives), titanium (for example titanates such as tetrabutyl orthotitanate or tetra(isopropyl) orthotitanate), zirconium (for example zirconates such as tetrabutyl orthozirconate or tetra(isopropyl) orthozirconate), antimony, cobalt, lead, aluminium (for example Al-triisopropylate), zinc, and mixtures thereof. In a particularly preferred embodiment, the catalyst used in polycondensation step 5 comprises a mixture of at least one titanium-based compound and at least one zirconium-based compound in which the Ti/(Ti + Zr) ratio by weight is within 0.01 and 0.4, preferably within 0.02 and 0.3.

In a preferred embodiment, the polycondensation catalyst based on titanium is a titanate advantageously selected from compounds having a general formula Ti(OR)₄ in which each R, independently of each other, is a ligand group comprising one or more carbon, oxygen, phosphorus, silicon and/or hydrogen atoms. Different ligand groups R may be present on one titanium atom, and these are preferably identical, so as to facilitate preparation of the titanate. In addition to this, 2 or more ligands R may derive from a single compound and may be chemically bound together in a way other than through the titanium (so-called multidentate ligands, such as for example triethanolamine, citric acid, glycolic acid, malic acid, succinic acid, ethandiamine). R is advantageously selected from H, triethanolamine, citric acid, glycolic acid, malic acid, succinic acid, 3-oxobutanoic acid, ethandiamine and linear or branched C₁-C₁₂ alkyl residues such as for example ethyl, propyl, n-butyl, n-pentyl, isopropyl, isobutyl, isopentyl, hexyl, 2-ethylhexyl. In a preferred embodiment, R is selected from C₁-C₁₂ alkyl, preferably C₁-C₈ alkyl, more preferably n-butyl.

Preparation of the titanates is known in the literature. Typically, they are prepared by reacting titanium tetrachloride and an alcohol of formula ROH in the presence of a base, such as for example ammonia, or via transesterification from other titanates. Commercial examples of titanates which can be used in the process according to this invention include the products Tyzor ^{®} TPT (tetra isopropyl titanate), Tyzor ^{®} TnBT (tetra n-butyl titanate) and Tyzor ^{®} TE (diisopropyl triethanolamine titanate).

In another preferred embodiment, the polycondensation catalyst based on zirconium is a zirconate advantageously selected from compounds having the general formula Zr(OR)₄ in which each R independently of each other is a ligand group comprising one or more carbon, oxygen, phosphorus, silicon and/or hydrogen atoms. As in the case of the titanates, different ligand groups R may be present on the same zirconium atom, but these are preferably identical so as to facilitate preparation of the zirconate. In addition to this 2 or more ligands R may derive from a single compound and may be chemically bound to each other by means other than via the zirconium (so-called multidentate ligands such as for example triethanolamine, citric acid, glycolic acid, malic acid, succinic acid, ethandiamine). R is advantageously selected from H, triethanolamine, citric acid, glycolic acid, malic acid, succinic acid, 3-oxobutanoic acid, ethandiamine and straight or branched C₁-C₁₂ alkyl residues such as for example ethyl, propyl, n-butyl, n-pentyl, isopropyl, isobutyl, isopentyl, hexyl, 2-ethylhexyl. In a preferred embodiment, R is selected from C₁-C₁₂ alkyl, preferably C₁-C₈ alkyl, more preferably n-butyl. Preparation of the zirconates is known in the literature. Commercial examples of zirconates which can be used in the process according to this invention include the products Tyzor ^{®} NBZ (tetra n-butyl zirconate), Tyzor NPZ (tetra n-propyl zirconate), IG-NBZ (tetra n-butyl zirconate).

Preferably the catalyst in polycondensation step 5 of the process of the invention comprises a mixture of at least one titanate and at least one zirconate, more preferably a mixture of tetra n-butyl titanate and tetra n-butyl zirconate.

In addition to titanium and zirconium compounds, the polycondensation catalyst may also comprise compounds containing phoshorous (for example phosphonic and phosphinic acids, organic phosphates and phosphites), silicates, organic or inorganic salts of alkali or alkaline earth metals.

The polycondensation catalyst may be fed to polycondensation step 5, or its various components may be fed separately to the reactor, or they may be premixed and fed to the reactor as a mixture. It is also possible for some of the components to be premixed and the composition of the catalyst to be adjusted subsequently, for example at the time when it is placed in contact with the oligomer product.

According to a preferred embodiment thereof, the catalyst containing titanium and/or zirconium used in step 1 is fed together with the oligomer resulting from step 1 to step 5 as the polycondensation catalyst or as a component thereof, if necessary adjusting the molar ratio between titanium and zirconium. In a particularly preferred embodiment, the catalyst for the polycondensation step 5 is the same used in step 1 of the process. Preferably polycondensation step 5 of the process is carried out in the presence of a total quantity of 80 - 500 ppm of titanium, or, in the case of catalysts containing other metals, the equivalent in molar terms of the said quantity of titanium, the said quantity of catalyst being determined with respect to the quantity of polyester which can be theoretically obtained by converting all the dicarboxylic acid fed to the reactor.

Polycondensation step 5 of the process of the invention is advantageously carried out by feeding the oligomer resulting from step 1 to the polycondensation reactor and allowing it to react in the presence of a catalyst at a temperature of 220 - 260 °C and at a pressure of ≤ 350 mbar. In one embodiment of the process of the invention an additional aliquot of a diol, preferably comprising 1,4-butanediol, in a quantity of 0 - 10% with respect to the total moles of dicarboxylic acids, may be added to step 5 in addition to the oligomer originating from step 1. Preferably the reaction time for polycondensation step 5 of the process is between 4 and 8 hours. Polycondensation step 5 of the process of the invention may be carried out in a single reactor or several reactors placed in series. Where not explicitly described otherwise, when reference is made to a step 5 performed in a reactor in this invention, configurations of step 5 of the process which comprise two or more reactors placed in series are also meant to be included. Reactors suitable for use in carrying out step 5 of the process are reactors capable of operating in the presence of high viscosity products such as polyesters, including for example reactors of the disc ring type or rotating-disc type possibly provided with strengthened stirrers.

In a preferred embodiment, step 5 of the process is carried out using a configuration of three reactors placed in series, in which a first vertical reactor with a stirrer (CSTR) operates at temperatures of 225 - 240 °C, pressures of 100-350 mbar and a residence time of 30 - 120 minutes, a second vertical reactor with stirrer (CSTR) operates at temperatures of 225 - 245 °C, pressures of 10 - 50 mbar with a residence time of 30 - 120 minutes and a third horizontal reactor with a disc stirrer operates at temperatures of 230 - 255 °C, pressures of 3 mbar or less and a residence time of 3 - 6 hours.

At the end of polycondensation step 5 a polyester preferably having Mn > 50000, more preferably Mn > 60000, is obtained. As far as the polydispersity index of the Mw/Mn molecular weights is concerned, this is preferably between 1.5 and 10, more preferably between 1.6 and 5 and even more preferably 1.8 - 2.7.

The terminal acid groups content of the polyesters obtained at the end of step 5 is preferably less than 70 meq/kg, more preferably < 50 meq/kg.

Preferably the polyesters obtained at the end of step 5 of the process according to this invention have an inherent viscosity (measured using an Ubbelohde viscometer for solutions in CHCl₃ at a concentration of 0.2 g/dl at 25°C) of more than 0.3 dl/g, preferably between 0.3 and 2 dl/g, more preferably between 0.4 and 1.1 dl/g, even more preferably 0.9 - 1.05 dl/g.

This process can be used for the combined production of THF and any polyester comprising repetitive 1,4-butylene dicarboxylate units, in which the 1,4-butanediol is condensed with a diacid which may also be in the form of a derivative thereof, such as for example an ester or salt. The said diacid may be either aliphatic or aromatic and is preferably selected from the group comprising aromatic dicarboxylic acids of the phthalic acid type, heterocyclic dicarboxylic aromatic compounds, saturated aliphatic dicarboxylic acids, unsaturated aliphatic dicarboxylic acids, their esters, salts and mixtures.

The aromatic dicarboxylic acids of the phthalic acid type are preferably terephthalic acid or isophthalic acid, more preferably terephthalic acid, their esters, salts and mixtures. The heterocyclic dicarboxylic aromatic compounds are preferably 2,5-furandicarboxylic acid, 2,4-furandicarboxylic acid, 2,3-furandicarboxylic acid, 3,4-furandicarboxylic acid, more preferably 2,5-furandicarboxylic acid, their esters, salts and mixtures.

The saturated aliphatic dicarboxylic acids are preferably selected from saturated C₂-C₂₄, preferably C₄-C₁₃, more preferably C₄-C₁₁, dicarboxylic acids, their C₁-C₂₄, preferably C₁-C₄, alkyl esters, their salts and mixtures. Preferably the saturated aliphatic dicarboxylic acids are selected from: succinic acid, 2-ethylsuccinic acid, glutaric acid, 2-methylglutaric acid, adipic acid, pimelic acid, suberic acid, azelaic acid, sebacic acid, undecanedioic acid, dodecanedioc acid, brassylic acid and their C₁-₂₄ alkyl esters.

The unsaturated aliphatic dicarboxylic acids are preferably selected from itaconic acid, fumaric acid, 4-methylene-pimelic acid, 3,4-bis (methylene) nonanedioic acid, 5-methylene-nonanedioic acid, their C₁-C₂₄, preferably C₁-C₄, alkyl esters, their salts and mixtures.

In one embodiment of this invention, the repetitive 1,4-butylene dicarboxylate units of the polyesters or copolyesters derive from the condensation of 1,4-butanediol with mixtures comprising two or more diacids, preferably of the type listed above.

In a preferred embodiment, the said 1,4-butylene dicarboxylate units derive from mixtures of aromatic diacids and aliphatic diacids comprising with respect to the total content of diacids:
- from 35 to 100% in moles, preferably 40-95%, of one or more aromatic diacids or heterocyclic dicarboxylic aromatic compounds, their esters or salts;
- from 0 to 65% in moles, preferably 5-60%, of one or more aliphatic diacids, their esters or salts.

In another preferred embodiment, the said 1,4-butylene dicarboxylate units derive from mixtures comprising at least two aromatic diacids, in turn comprising with respect to the total content of aromatic diacids:
- from 1 to 99% in moles, preferably from 5 to 95% and more preferably from 10 to 80%, of terephthalic acid, its esters or its salts;
- from 99 to 1% in moles, preferably from 95 to 5% and more preferably from 90 to 20% of 2,5-furandicarboxylic acid, its esters or salts.

In another preferred embodiment of this invention, the said 1,4-butylene dicarboxylate units derive from mixtures comprising at least two saturated aliphatic diacids in turn comprising with respect to the total content of aliphatic diacids at least 50% in moles, preferably more than 60% in moles, more preferably more than 65% in moles of one or more saturated aliphatic diacids selected from the group comprising succinic acid, adipic acid, azelaic acid, sebacic acid, brassylic acid, their C₁-C₂₄, preferably C₁-C₄, esters and mixtures thereof.

In the case of copolyesters, these preferably contain 70% in moles, more preferably 80% in moles, of 1,4-butylene dicarboxylate units. In addition to the 1,4-butylene dicarboxylate units the said copolyesters preferably comprise alkylene dicarboxylate units in which the alkylene group derives from the condensation of one or more diols other than 1,4-butanediol preferably selected from the group comprising saturated aliphatic diols and unsaturated aliphatic diols, aromatic diols and mixtures thereof. More preferably the saturated aliphatic diols are selected from the group comprising 1,2-ethanediol, 1,2-propanediol, 1,3-propanediol, 1,5-pentanediol, 1,6-hexanediol, 1,7-heptanediol, 1,8-octanediol, 1,9-nonanediol, 1,10-decanediol, 1,11-undecanediol, 1,12-dodecanediol, 1,13-tridecanediol, 1,4-cyclohexanedimethanol, neopentylglycol, 2-methyl-1,3-propanediol, dianhydrosorbitol, dianhydromannitol, dianhydroiditol, cyclohexanediol, cyclohexanedimethanol, dialkylene glycol and polyalkylene glycols having a molecular weight of 100 - 4000, such as for example polyethylene glycol, polypropylene glycol and their mixtures. The unsaturated aliphatic diols are preferably selected from the group comprising cis 2-butene-1,4-diol, trans 2-butene-1,4-diol, 2-butyne-1,4-diol, cis 2-pentene-1,5-diol, trans 2-pentene-1,5-diol, 2-pentyne-1,5-diol, cis 2-hexene- 1,6-diol, trans 2-hexene-1,6-diol, 2-hexyne-1,6-diol, cis 3-hexene-1,6-diol, trans 3-hexene-1,6-diol, 3-hexyne-1,6-diol and mixtures thereof.

The aromatic diols are instead more preferably selected from the group consisting of 2,5-furandimethanol, 2,4-furandimethanol, 2,3-furandimethanol, 3,4-furandimethanol, more preferably 2,5-furandimethanol and their mixtures.

As far as the diacids in the repetitive units of the copolymers which are other than 1,4-butylene dicarboxylate units are concerned, these are preferably selected from the diacids mentioned above for the 1,4-butylene dicarboxylate unit itself.

In addition to the 1,4-butylene dicarboxylate units and any different alkylene dicarboxylate units, the polyesters and copolyesters according to this invention preferably comprise repetitive units deriving from at least one hydroxy acid in a quantity of between 0 and 49%, preferably between 0 and 30% in moles with respect to the total moles of the dicarboxylic component. Examples of convenient hydroxy acids are glycolic, hydroxybutyric, hydroxycaproic, hydroxyvaleric, 7-hydroxyheptanoic, 8-hydroxycaproic, 9-hydroxy nonanoic and lactic acid or lactide. The hydroxy acids may be inserted in a chain as such or may also be first caused to react with diacids or diols. In the process according to this invention, the said hydroxy acids are advantageously added in step 1 of this process (being part of the initial mixture).

Quantities of not more than 10% in moles, with respect to the total moles of the total dicarboxylic components, of long molecules with two functional groups, including functional groups not in a terminal position, may also be present in the polyesters resulting from step 5. Examples are dimer acids, ricinoleic acid and acids incorporating epoxy groups and also polyoxyethylenes having a molecular weight of between 200 and 10000. In the process according to this invention, said long molecules with two functional groups are advantageously added during step 1 of the process (even as part of the starting mixture).

Diamines, amino acids and amino alcohols may also be present in percentages up to 30% in moles with respect to the total moles of the total dicarboxylic component.

In the process according to this invention, said diamines, amino acids and amino alcohols are advantageously added in step 1 of the process (even as part of the starting mixture).

One or more molecules having multiple functional groups in quantities of between 0.1 and 3% in moles with respect to the total moles of the dicarboxylic component (including any hydroxy acids) may also be added during step 1 of the process in order to obtain branched products. Examples of these molecules are glycerol, pentaerythritol, trimethylolpropane, citric acid, dipentaerythritol, acid triglycerides, polyglycerols.

Preferably the polyesters resulting from step 5 are biodegradable. In the meaning of this invention by biodegradable polyesters are meant biodegradable polyesters according to standard EN 13432.

Typical examples of polyesters comprising 1,4-butylene dicarboxylate units according to this invention are poly(1,4-butylene succinate), poly(1,4-butylene adipate), poly(1,4-butylene azelate), poly(1,4-butylene sebacate), poly(1,4-butylene adipate-co-1,4-butylene succinate), poly(1,4-butylene azelate-co-1,4-butylene succinate), poly(1,4-butylene sebacate-co-1,4-butylene succinate), poly(1,2-ethylene adipate-co-1,4-butylene succinate), poly(1,2-ethylene azelate-co-1,4-butylene succinate), poly(1,2-ethylene sebacate-co-1,4-butylene succinate), poly(1,4-butylene adipate-co-1,2-ethylene succinate), poly(1,4-butylene azelate-co-1,2-ethylene succinate), poly(1,4-butylene sebacate-co-1,2-ethylene succinate), poly(1,4-butylene adipate-co-1,4-butylene terephthalate), poly(1,4-butylene sebacate-co-1,4-butylene terephthalate), poly(1,4-butylene azelate-co-1,4-butylene terephthalate), poly(1,4-butylene brassylate-co-1,4-butylene terephthalate), poly(1,4-butylene succinate-co-1,4-butylene terephthalate), poly(1,2-ethylene adipate-co-1,4-butylene terephthalate), poly(1,2-ethylene sebacate-co-1,4-butylene terephthalate), poly(1,2-ethylene azelate-co-1,4-butylene terephthalate), poly(1,2-ethylene brassylate-co-1,4-butylene terephthalate), poly(1,2-ethylene succinate-co-1,4-butylene terephthalate), poly(1,4-butylene adipate-co-1,2-ethylene terephthalate), poly(1,4-butylene sebacate-co-1,2-ethylene terephthalate), poly(1,4-butylene azelate-co-1,2-ethylene terephthalate), poly(1,4-butylene brassylate-co-1,2-ethylene terephthalate), poly(1,4-butylene succinate-co-1,2-ethylene terephthalate), poly(1,4-butylene adipate-co-1,4-butylene 2,5-furandicarboxylate), poly(1,4-butylene sebacate-co-1,4-butylene 2,5-furandicarboxylate), poly(1,4-butylene azelate-co-1,4-butylene 2,5-furandicarboxylate), poly(1,4-butylene brassylate-co-1,4-butylene 2,5-furandicarboxylate), poly(1,4-butylene succinate-co-1,4-butylene 2,5-furandicarboxylate), poly(1,2-ethylene adipate-co-1,4-butylene 2,5-furandicarboxylate), poly(1,2-ethylene sebacate-co-1,4-butylene 2,5-furandicarboxylate), poly(1,2-ethylene azelate-co-1,4-butylene 2,5-furandicarboxylate), poly(1,2-ethylene brassylate-co-1,4-butylene 2,5-furandicarboxylate), poly(1,2-ethylene succinate-co-1,4-butylene 2,5-furandicarboxylate), poly(1,4-butylene adipate-co-1,2-ethylene 2,5-furandicarboxylate), poly(1,4-butylene sebacate-co-1,2-ethylene 2,5-furandicarboxylate), poly(1,4-butylene azelate-co-1,2-ethylene 2,5-furandicarboxylate), poly(1,4-butylene brassylate-co-1,2-ethylene 2,5-furandicarboxylate), poly(1,4-butylene succinate-co-1,2-ethylene 2,5-furandicarboxylate), poly(1,4-butylene terephthalate), poly(1,4-butylene 2,5-furandicarboxylate), poly(1,2-ethylene terephthalate-co-1,4-butylene terephthalate), poly(1,3-propylene terephthalate-co-1,4-butylene terephthalate), poly(1,4-butylene terephthalate-co-1,4-butylene 2,5-furandicarboxylate), poly(1,2-ethylene terephthalate-co-1,4-butylene 2,5-furandicarboxylate), poly(1,3-propylene terephthalate-co-1,4-butylene 2,5-furandicarboxylate), poly(1,2-ethylene terephthalate-co-1,4-butylene 2,5-furandicarboxylate) poly(1,4-butylene terephthalate-co-1,2-ethylene 2,5-furandicarboxylate), their block or random copolymers.

In a preferred embodiment, the polyesters comprising 1,4-butylene dicarboxylate units according to this invention are preferably selected from poly(1,4-butylene succinate), poly(1,4-butylene adipate-co-1,4-butylene terephthalate), poly(1,4-butylene azelate-co-1,4-butylene terephthalate), poly(1,4-butylene adipate-co-1,4-butylene 2,5-furandicarboxylate), poly(1,4-butylene azelate-co-1,4-butylene 2,5-furandicarboxylate), poly(1,4-butylene terephthalate), poly(1,4-butylene 2,5-furandicarboxylate).

Depending upon the specific properties desired for the polyester, the process according to this invention may provide for at least a subsequent step 6 after polycondensation step 5 comprising:
- the chain extension, preferably through the use of peroxides, divinyl ethers, bisoxazolines, polyepoxides, di- and poly-isocyanates, carbodiimides, dianhydrides or mixtures thereof;
   and/or
- mixing with other polymers and/or additives.

In a preferred embodiment, the process according to the invention comprises a step 6 in which the polyester obtained at the end of step 5
i. is allowed to react, preferably in an extruder, at a temperature of 150 - 200 °C with 0.05 - 0.8% by weight, preferably 0.15 - 0.5% by weight, with respect to the weight of the polyester obtained in step 5, with at least one cross-linking agent and/or chain extender comprising at least one compound having two and/or multiple functional groups including isocyanate, peroxide, carbodiimide, isocyanurate, oxazoline, epoxy, anhydride, or divinyl ether groups and mixtures thereof;
   and/or
ii. is mixed, preferably in an extruder, at a temperature of 150 - 250 °C with 10 - 70% by weight, with respect to the weight of the polyester obtained in step 5, with one or more polymers selected from the group consisting of hydroxy acid polyesters, polyolefins, aromatic polyesters not comprising 1,4-butylene dicarboxylate units, polyester- and polyether-urethanes, polyurethanes, polyamides, polyamino acids, polyethers, polyureas, polycarbonates and/or one or more additives selected from fillers, plasticisers, UV stabilisers, lubricants, nucleating agents, surfactants, antistatic agents, pigments, flame retardants, compatibilising agents, polyphenols, reinforcing fillers, coupling agents, antioxidants, mould-preventing agents, waxes and process coadjuvants.

Preferably the cross-linking and/or chain extending agent comprises at least one compound having two and/or multiple functional groups incorporating isocyanate groups. More preferably the cross-linking agent and/or chain extender comprises at least 25% by weight of one or more compounds having two and/or multiple functional groups incorporating isocyanate groups, or, even more preferably, at least 75% by weight of one or more compounds having two and/or multiple functional groups incorporating isocyanate groups. Particularly preferred are mixtures of compounds with two and/or multiple functional groups including isocyanate groups with compounds having two and/or multiple functional groups including epoxide groups..

The compounds having two and multiple functional groups incorporating isocyanate groups are preferably selected from p-phenylene diisocyanate, 2,4-toluene diisocyanate, 2,6-toluene diisocyanate, 4,4-diphenylmethane-diisocyanate, 1,3-phenylene-4-chloro diisocyanate, 1,5-naphthalene diisocyanate, 4,4-diphenylene diisocyanate, 3,3'-dimethyl-4,4-diphenylmethane diisocyanate, 3-methyl-4,4'-diphenylmethane diisocyanate, diphenylester diisocyanate, 2,4-cyclohexane diisocyanate, 2,3-cyclohexane diisocyanate, 1-methyl 2,4-cyclohexyl diisocyanate, 1-methyl 2,6-cyclohexyl diisocyanate, bis-(isocyanate cyclohexyl) methane, 2,4,6-toluene triisocyanate, 2,4,4-diphenylether triisocyanate, polymethylene-polyphenyl-polyisocyanate, methylene diphenyl diisocyanate, triphenylmethane triisocyanate, 3,3'-ditolylene-4,4-diisocyanate, 4,4'-methylene bis (2-methyl-phenyl isocyanate), hexamethylene diisocyanate, 1,3-cyclohexylene diisocyanate, 1,2-cyclohexylene diisocyanate and mixtures thereof. In a preferred embodiment, the compound incorporating isocyanate groups is 4,4-diphenylmethane diisocyanate.

As far as the compounds having two and multiple functional groups incorporating peroxide groups are concerned, these are preferably selected from benzoyl peroxide, lauroyl peroxide, isononanoyl peroxide, di-(t-butylperoxyisopropyl)benzene, for example 1,3- and 1,4-di(-2-(t-butylperoxy)prop-2-yl)benzene, t-butyl peroxide, dicumyl peroxide, alpha, alpha'-di(t-butylperoxy)diisopropylbenzene, 2,5-dimethyl-2,5-di(t-butylperoxy)hexane, t-butyl cumyl peroxide, di-t-butylperoxide, 2,5-dimethyl-2,5-di(t-butylperoxy)hex-3-yne, di(4-t-butylcyclohexyl)peroxy dicarbonate, dicetyl peroxydicarbonate, dimyristyl peroxydicarbonate, 3,6,9-triethyl-3,6,9-trimethyl-1,4,7-triperoxonan, di(2-ethylhexyl) peroxydicarbonate and mixtures thereof.

The compounds having two and multiple functional groups incorporating carbodiimide groups are preferably selected from poly(cyclooctylene carbodiimide), poly(1,4-dimethylencyclohexylene carbodiimide), poly(cyclohexylene carbodiimide), poly(ethylene carbodiimide), poly(butylene carbodiimide), poly(isobutylene carbodiimide), poly(nonylene carbodiimide), poly(dodecylene carbodiimide), poly(neopentylene carbodiimide), poly(1,4-dimethylene phenylene carbodiimide), poly(2,2',6,6', tetraisopropyldiphenylene carbodiimide) (Stabaxol^{®} D), poly(2,4,6-triisopropyl-1,3-phenylene carbodiimide) (Stabaxol^{®} P-100), poly(2,6 diisopropyl-1,3-phenylene carbodiimide) (Stabaxol^{®} P), poly(tolyl carbodiimide), poly(4,4'- diphenylmethane carbodiimide), poly(3,3'-dimethyl-4,4'-biphenylene carbodiimide), poly(p-phenylene carbodiimide), poly(m-phenylene carbodiimide), poly(3,3'-dimethyl-4,4'-diphenylmethane carbodiimide), poly(naphthylene carbodiimide), poly(isophorone carbodiimide), poly(cumene carbodiimide), p-phenylene bis(ethylcarbodiimide), 1,6-hexamethylene bis(ethylcarbodiimide), 1,8-octamethylene bis(ethylcarbodiimide), 1,10-decamethylene bis(ethylcarbodiimide), 1,12 dodecamethylene bis(ethylcarbodiimide) and their mixtures.

Examples of compounds with two and multiple functional groups incorporating epoxy groups which may advantageously be used according to this invention are the polyepoxides for example epoxylated oils and/or styrene - glycidyl ether-methylmethacrylate, glycidyl ether-methylmethacrylate included in a range of molecular weights between 1000 and 10000 and having an epoxy number per molecule in the range from 1 to 30 and preferably 5 to 25, and epoxides selected from the group comprising: diethylene glycol diglycidyl ether, polyethylene glycol diglycidyl ether, glycerol polyglycidyl ether, diglycerol polyglycidyl ether, 1,2-epoxybutane, polyglycerol polyglycidyl ether, isoprene diepoxide and cycloaliphatic diepoxides, 1,4-cyclohexanedimethanol diglycidyl ether, glycidyl 2-methylphenyl ether, glycerol propoxylate triglycidyl ether, 1,4-butanediol glycidyl ether, sorbitol polyglycidyl ether, glycerol diglycidyl ether, meta-xylene diamine tetraglycidyl ether, the diglycidyl ether of bisphenol A, and their mixtures.

In addition to the compounds having two and multiple functional groups incorporating isocyanate, peroxide, carbodiimide, isocyanurate, oxazoline, epoxy, anhydride and divinylether groups, other catalysts may be used to increase the reactivity of the reactive groups. Salts of fatty acids, for example calcium and/or zinc stearates, are preferably used in the case of polyepoxides.

In a particularly preferred embodiment of the invention, the cross-linking agent and/or chain extender comprises compounds including isocyanate groups (preferably 4,4-diphenylmethane-diisocyanate), epoxy groups (preferably styrene-glycidyl ether-methylmethacrylate, glycidyl ether methylmethacrylate), peroxide groups (preferably 1,3-1,4-di(-2-(t-butylperoxy)prop-2-yl)benzene), and mixtures thereof.

Among the hydroxy acid polyesters those preferred are: polyesters of lactic acid, poly-ε-caprolactone, poly hydroxybutyrate, poly hydroxybutyrate-valerate, poly hydroxybutyrate propanoate, poly hydroxybutyrate-hexanoate, poly hydroxybutyrate-decanoate, poly hydroxybutyrate-dodecanoate, poly hydroxybutyrate-hexadecanoate, poly hydroxybutyrate-octadecanoate, poly 3-hydroxybutyrate, 4-hydroxybutyrate.

Preferably the said hydroxy acid polyesters comprise at least 80% by weight of one or more polyesters of lactic acid with respect to the total weight of hydroxy acid polyesters. The said lactic acid polyesters are preferably selected from the group comprising poly-L-lactic acid, poly-D-lactic acid, stereo poly-D-L lactic acid complex, copolymers comprising more than 50% in moles of the said lactic acid polyesters or their mixtures. Particularly preferred are polyesters of lactic acid comprising at least 95% by weight of repetitive units deriving from L-lactic or D-lactic acid or combinations thereof having an molecular weight Mw of more than 50,000 and a shear viscosity of between 50 and 700 Pas, preferably between 80 and 500 Pas (measured according to standard ASTM D3835 at T = 190°C, shear rate = 1000 s⁻¹, D = 1 mm, L/D = 10), such as for example Ingeo^{™} Biopolymers 4043D, 3251D and 6202D.

Preferably the polyester mixtures obtained from step 5 of the process according to the invention with the hydroxy acid polyesters described above are characterised by a said hydroxy acid polyester content which varies within the range 5 to 80% by weight, more preferably between 10 and 70% by weight with respect to the sum of the weights of the polyesters obtained from the process according to this invention and the latter respectively.

Among the polyolefins, those preferred are polyethylene, polypropylene, their copolymers, polyvinyl alcohol, polyvinyl acetate, polyethylvinyl acetate and polyethylene vinyl alcohol.

Among the aromatic polyesters those preferred are PET, PBT, PTT, in particular having a renewable content > 30% and polyalkylene furandicarboxylates. Among the latter those particularly preferred are poly(1,2-ethylene 2,5-furandicarboxylate), poly(1,3-propylene 2,5-furandicarboxylate), poly(1,4-butylene 2,5-furandicarboxylate) and their mixtures.

Examples of polyamides are polyamide 6 and 6,6, polyamide 9 and 9,9, polyamide 10 and 10,10, polyamide 11 and 11,11, polyamide 12 and 12,12 and their combinations of the 6/9, 6/10, 6/11 and 6/12 type.

The polycarbonates may be polyethylene carbonates, polypropylene carbonates, polybutylene carbonates, their mixtures and copolymers.

The polyethers may be polyethylene glycols, polypropylene glycols, polybutylene glycols, their copolymers and their mixtures having molecular weights from 70000 to 500000.

Preferably the mixtures of polyesters obtained from the process according to this invention with polymers selected from polyolefins, aromatic polyesters, polyester- and polyether-urethanes, polyurethanes, polyamides, polyamino acids, polyethers, polyureas, polycarbonates and mixtures thereof comprise 5-80% by weight, more preferably 10-60% by weight of the said polymers with respect to the sum of the weights of the polyesters obtained from the process according to this invention and the said polymers respectively.

Fillers are preferably selected from the group comprising kaolin, barytes, clay, talc, calcium and magnesium, iron and lead carbonates, aluminium hydroxide, diatomaceous earth, aluminium sulphate, barium sulphate, silica, mica, titanium dioxide, wollastonite, starch, cellulose, chitin, chitosan, alginates, proteins such as gluten, zein, casein, collagens, gelatin, natural rubbers, rosinic acid and their derivatives and mixtures thereof.

By the term starch is here meant starch of all types, that is flour, native starch, hydrolysed starch, destructurized starch, gelatinised starch, plasticised starch, thermoplastic starch, biofillers comprising complex starch or mixtures thereof. Particularly suitable according to the invention are starches such as potato, maize, tapioca and pea starch.

Starches which are capable of being easily destructurized and which have high initial molecular weights, such as for example potato and maize starches, have proved to be particularly advantageous.

Starch and cellulose may be present as such or in chemically modified form, such as for example in the form of starch or cellulose esters having a degree of substitution of between 0.2 and 2.5, hydroxypropylate starch, starch modified with fatty chains, or as cellophane.

With respect to destructurized starch reference is made to the teachings of EP-0 118 240 and EP-0 327 505, which are incorporated herein by reference. Destructurized starch according this invention is starch which has been worked in such a way as to substantially not present the so-called "Maltese crosses" under a polarised light optical microscope and the so-called "ghosts" under a phase contrast optical microscope.

Advantageously, destructurization of the starch is carried out through an extrusion process at temperatures of between 110 and 250°C, preferably 130 - 180°C, preferably at pressures between 0.1 and 7 MPa, preferably 0.3 - 6 MPa, preferably providing a specific energy of more than 0.1 kWh/kg during the said extrusion. Said destructurization may be carried out during either step 6 of the process according to the invention or in a separate step, then feeding the starch already in destructurized form to step 6 of the process.

Destructurization of starch preferably takes place in the presence of 1-40% by weight with respect to the weight of the starch of one or more plasticisers selected from water and polyols having from 2 to 22 carbon atoms. As far as the water is concerned, this may also be that which is naturally present in the starch. Among the polyols, polyols having from 1 to 20 hydroxyl groups containing from 2 to 6 carbon atoms, their ethers, thioethers and organic and inorganic esters are preferred. Examples of polyols are glycerine, diglycerol, polyglycerol, pentaerythritol, polyglycerol ethoxylate, ethylene glycol, polyethylene glycol, 1,2-propanediol, 1,3-propanediol, 1,4-butanediol, neopentylglycol, sorbitol monoacetate, sorbitol diacetate, sorbitol monoethoxylate, sorbitol diethoxylate, and mixtures thereof. In a preferred embodiment, the starch is destructurized in the presence of glycerol or a mixture of plasticisers comprising glycerol, more preferably comprising between 2 and 90% by weight of glycerol. Preferably the destructurized starch comprises between 1 and 40% by weight with respect to the weight of the starch of plasticisers selected from those listed above.

Compositions comprising destructurized starch are particularly preferred.

Preferably the starch in the composition is present in the form of particles having a circular or elliptical cross-section having a mean arithmetic diameter of less than 1 micron measured in relation to the major axis of the particle and preferably less than 0.5 µm mean diameter.

As far as the cellulose is concerned this will be present for example in the form of cellulose fibres or wood flour.

Advantageously more than one filler may be used in the compositions according to this invention. Particularly preferred are mixtures concerning starch and at least one other filler.

As far as plasticisers are concerned, in addition to any plasticisers which are preferably used for preparation of the destructurized starch and described above, one or more plasticisers selected from the group comprising phthalates, such as for example diisononyl phthalate, trimellitates, such as for example esters of trimellitic acid with C₄-C₂₀ mono alcohols preferably selected from the group comprising n-octanol and n-decanol and aliphatic esters having the following structures:

R₁-O- C(O)-R₄-C(O)-[-O-R₂-O-C(O)-R₅-C(O)-]ₘ-O-R₃

in which
R₁ is selected from one or more of the groups comprising H, linear and branched saturated and unsaturated C₁-C₂₄ alkyl residues, polyol residues esterified with C₁-C₂₄ monocarboxylic acids;
R₂ comprises -CH₂-C(CH₃)₂-CH₂- groups and C₂-C₈ alkylene groups, and comprises at least 50% in moles of the said -CH₂-C(CH₃)₂-CH₂- groups;
R₃ is selected from one or more of the groups comprising H, linear and branched saturated and unsaturated C₁-C₂₄ alkyl residues, polyol residues esterified with C₁-C₂₄ monocarboxylic acids;
R₄ and R₅ are the same or different, comprising one or more C₂-C₂₂, preferably C₂-C₁₁, more preferably C₄-C₉, alkylenes and comprise at least 50% in moles of C₇ alkylenes; and
m is a number of between 1 and 20, preferably 2 and 10, more preferably 3 and 7.

Preferably in the said esters at least one of the R₁ and/or R₃ groups comprises polyol residues partly or fully esterified with at least one C₁-C₂₄ monocarboxylic acid selected from the group consisting of stearic acid, palmitic acid, 9-ketostearic acid, 10-ketostearic acid and mixtures thereof preferably in quantities ≥ 10% in moles, more preferably ≥ 20%, even more preferably ≥ 25% in moles with respect to the total quantity of R₁ and/or R₃ groups. Examples of aliphatic esters of this type are described in Italian Patent Application MI2014A000030 and in PCT Applications PCT/EP2015/050336 and PCT/EP2015/050338.

The plasticisers are preferably used in quantities of between 0.2 and 20% by weight, more preferably between 0.5 and 10% by weight, with respect to the total weight of the composition. The lubricants are preferably selected from esters and metal salts of fatty acids such as for example zinc stearate, calcium stearate, aluminium stearate and acetyl stearate.

Preferably, when used, said lubricants are added in quantities up to 1% by weight, more preferably up to 0.5% by weight, with respect to the total weight of the composition.

Examples of nucleating agents include the sodium salt of saccharin, calcium silicate, sodium benzoate, calcium titanate, boron nitride, talc, zinc stearate, and low molecular weight PLA. These additives are preferably added in quantities up to 10% by weight and more preferably between 2 and 6% by weight with respect to the total weight of the composition.

Pigments may also be added if necessary, for example clays, copper phthalocyanine, titanium dioxide, iron silicates, oxides and hydroxides, carbon black, and magnesium oxide. These additives will preferably be added up to 10% by weight.

As far as the polyphenols are concerned, these are preferably selected from the group comprising lignin, silybin, silydianin, isosilybin and silychristin and mixtures thereof, and are present in quantities of preferably between 0.5 and 7% by weight with respect to the total weight of the composition. In a preferred embodiment, the polyphenol of plant origin advantageously comprises a mixture comprising silybin, silydianin, isosilybin and silychristin. The said mixture may advantageously be obtained by alcoholic extraction from the de-oiled cake of milk thistle (*Silybum marianum*) seeds and is commonly known in commerce by the name of silymarin.

The polyesters obtained from the process according to this invention are particularly suitable for use alone or in a mixture with other polymers in many practical applications for the manufacture of products such as for example films, fibres, non-woven fabrics, sheets, moulded articles, thermoformed articles, blown and expanded articles and laminated articles, including using the extrusion coating technique.

Examples of products comprising the polyesters obtained by the process according to this invention are:
- films, both mono and bi-oriented, and multilayer film with other polymer materials;
- film for use in the agricultural sector as a film for mulching;
- stretch film including clingfilm for foodstuffs, for bales in agriculture and for enclosing wastes;
- bags and linings for organic collection such as the collection of food waste and grass cuttings;
- thermoformed food packaging, both single layer and multilayer, such as for example containers for milk, yoghurt, meat, beverages;
- coatings obtained by the extrusion coating technique;
- multilayer laminates with layers of paper, plastics materials, aluminium and metallic films;
- expanded or expandable beads for the production of parts formed by sintering;
- expanded and semi-expanded products including expanded blocks formed from pre-expanded particles;
- expanded sheets, thermoformed expanded sheets, containers obtained therefrom for food packaging;
- containers in general for fruit and vegetables;
- composites with gelatinised, destructurized and/or complexed starch, natural starch, flours, other fillers of natural, plant or inorganic origin as fillers;
- fibres, microfibres, composite fibres with a core comprising rigid polymers such as PLA, PET, PTT, etc., and an outer shell of the material according to the invention, dablens composite fibres, fibres having various cross-sections from round to multilobate, flock, woven and non-woven or spun-bonded or thermobonded fibres for the health, hygiene, agriculture and clothing sectors.

They may also be used in applications as a replacement for plasticised PVC.

The invention will now be illustrated with some examples which are to be understood to be by way of examples and do not limit the scope of protection of this patent application.

### EXAMPLES

### EXAMPLE 1 - Process for the combined production of THF and a poly(1,4-butylene adipate-co-1,4-butylene terephthalate)

1640 kg/h of terephthalic acid, 1666 kg/h of adipic acid, 2055 kg/h of 1,4-butanediol, 0.625 kg/h of glycerine were mixed at 60°C in a vertical vessel with a stirrer and subsequently continuously fed to a first vertical esterification reactor with a stirrer (CSTR) provided with an oil heat-transfer agent jacket and heated by means of an oil heat-transfer coil, to which were added 1.1 kg/h of Tyzor TE and 1825 kg/h of 1,4-butanediol, operating at 205°C and 60 mBarg (1060 mBar) with a residence time of 6 hours.

A vapour phase containing THF, water and unreacted 1,4-butanediol was continuously separated off from the reaction mixture in the first esterification reactor and combined with the vapour phase separated from the second esterification reactor and then passed to a distillation column with structured packing, three plates and a coil at the base of the column heated with heat transfer oil. The column operated at 25 mbarg (1025 mbar), bottom T 170°C and head T of 96°C, reflux ratio 0.6, from which 1500 l/h of a light fraction containing 35% THF and 20 ppm of 1,4-butanediol was separated off from the head and 3390 kg/h of a heavy fraction containing 95% of 1,4-butanediol was separated off from the bottom.

The reaction mixture from the first esterification reactor was then continuously transferred to a second vertical esterification reactor with a stirrer (CSTR), a heat-transfer oil jacket heated by means of a heat-transfer oil coil operating at 225°C and 65 mBarg (1065 mbar) with a residence time of 3.3 hours, from which vapour containing THF, water and 1,4-butanediol was continuously discharged; the latter was combined with the vapour phase separated from the first esterification reactor before being sent to the distillation column, and 4635 kg/h of an oligomer product having Mn < 1500, an inherent viscosity of 0.07 dl/g and an acidity of 400 meq/kg was discharged.

The said oligomer product was then continuously fed to a first vertical polycondensation reactor with a stirrer (CSTR), a heat-transfer oil jacket heated by means of a coil with heat transfer oil operating at 237°C and 100 mBarg with a residence time of 1.4 hours. The product so obtained was then transferred continuously to a second vertical reactor with a stirrer (CSTR), the heat-transfer oil jacket heated by means of a heat-transfer oil coil operating at 242°C and 18 mBarg with a residence time of 1.4 hours, and to this was also fed 5.1 kg/h of Tyzor TnBt, and subsequently from this to a third horizontal reactor with a disc stirrer operating at between 244 and 254°C and 1.2 mBar with a residence time of 3 hours. At the end of the reaction the polyester obtained had an Mn of 75000, an inherent viscosity of 1.0 dl/g and an acidity of 40 meq/kg.

## Claims

1. Combined process for the production of tetrahydrofuran and polyesters comprising 1,4-butylene dicarboxylate units, comprising the steps of:
1. subjecting a mixture comprising 1 ,4-butanediol and at least one dicarboxylic acid, an ester, a salts or a derivative thereof to esterification and/or transesterification to produce an oligomer and a vapour phase comprising tetrahydrofuran and 1,4-butanediol;
2. separating the vapour phase comprising tetrahydrofuran and 1 ,4-butanediol from the oligomer;
3. distilling the vapour phase in a distillation column comprising at least one plate section and at least one packed section;
4. separating a light fraction comprising tetrahydrofuran and less than 1% by weight of 1 ,4-butanediol from a heavy fraction comprising 1 ,4-butanediol;
5. polycondensing the oligomer obtained from step 1;
wherein at least some of the components of the mixture of step 1 are premixed, before being delivered to the reactor.

2. Combined process according to claim 1, wherein said components are premixed at a temperature below 70 °C.

3. Combined process according to any of claims 1-2, wherein the esterification/transesterification catalyst is fed directly to step 1.

4. Combined process according to any of claims 1-2, wherein the esterification/transesterification catalyst is first dispersed in an aliquot of one or more dicarboxylic acids, their esters or salts, and/or of diols to promote a homogenous dispersion in the reaction medium.

5. Combined process according to any of claims 1 to 4, wherein the step 1 is carried out in two or more esterification reactors arranged in series.

6. Combined process according to claim 5, wherein said step 1 is carried out in two esterification reactors placed in series, in which the first reactor operates at a temperature of 190 - 230°C and a pressure of 0.7 - 1.5 bar, with a residence time of 3 - 7 hours, and the second reactor operates at a temperature of 205 - 250°C and a pressure of 07 - 1.5 bar, with a residence time of 1 - 3.5 hours.

7. Combined process according to any of claims 1-6, wherein the polycondensation step 5 is carried out in the presence of a catalyst.

8. Combined process according to claim 7, wherein the catalyst is the same of that used in step 1.

9. Combined process according to claim 7, wherein the catalyst used in step 1 and the oligomer resulting from the esterification and/or transesterification reaction are fed to the polycondensation step 5 of the process.

10. Combined process according to any of claims 1 to 9, wherein the part of the distillation column in step 3 provided with at least one plate constitutes the 15-35% of the column, and the part provided with packing constitutes the 85-65% of the distillation column.

11. Combined process according to any of claims 1 to 10, wherein the distillation column in step 3 operates at pressures between 500 mbar and 1200 mbar with bottom temperatures of between 150°C and 195°C, head temperatures between 80°C and 98°C and using a reflux ratio of between 0.5 and 2.

12. Combined process according to any one of claims 1 to 11, wherein an oligomer product having Mn < 5000 as measured using Gel Permeation Chromatography (GPC) as defined in the specification, an inherent viscosity of 0.03 - 0.15 dl/g as measured using an Ubbelhode viscometer for solutions in CHCl₃ at a concentration of 0.2 g/dl at 25°C, and a terminal acidity of 400 meq/kg or below is obtained at the end of step 1.

13. Combined process according to any one of claims 1 to 12, wherein said step 5 is carried out at a temperature of 220 - 260°C and a pressure of 350 mbar or below.

14. Combined process according to any one of claims 1 to 13, wherein said step 5 is carried out in the presence of a total quantity of catalyst based on titanium and zirconium containing 80-500 ppm of metal with respect to the quantity of polyester which can theoretically be obtained by converting all the dicarboxylic acid fed to the process.

15. Combined process according to any one of claims 1 to 14, comprising one or more chain extension and/or reactive extrusion steps after step 5.

16. Combined process according to any one of claims 1 to 15, in which said 1,4-butylene dicarboxylate units of said polyesters derive from mixtures of aromatic diacids and aliphatic diacids comprising, with respect to the total content of diacids:
- from 35 to 100% by moles, preferably 40-95%, of one or more aromatic diacids or heterocyclic dicarboxylic aromatic compounds, their esters or salts;
- from 0 to 65% by moles, preferably 5-60%, of one or more aliphatic diacids, their esters or salts.

17. Combined process according to any one of claims 1 to 16, in which said polyesters comprising 1,4-butylene dicarboxylate units derive from mixtures comprising at least two aromatic diacids, in turn comprising with respect to the total content of aromatic diacids:
- from 1 to 99% by moles, preferably from 5 to 95% and more preferably from 10 to 80%, of terephthalic acid, its esters or its salts;
- from 99 to 1 % by moles, preferably from 95 to 5% and more preferably from 90 to 20% of 2,5-furandicarboxylic acid, its esters or salts.

18. Combined process according to any one of claims 1 to 17, in which said polyesters comprising 1,4-butylene dicarboxylate units are selected from the group consisting of poly(1,4-butylene succinate), poly(1,4-butylene adipate), poly(1,4-butylene azelate), poly(1,4-butylene sebacate), poly(1,4-butylene adipate-co-1,4-butylene succinate), poly(1,4-butylene azelate-co-1,4-butylene succinate), poly(1,4-butylene sebacate-co-1.4-butylene succinate), poly(1,2-ethylene adipate-co-1,4-butylene succinate), poly(1,2-ethylene azelate-co-1,4-butylene succinate), poly(1,2-ethylene sebacate-co-1,4-butylene succinate), poly(1,4-butylene adipate-co-1,2-ethylene succinate), poly(1,4-butylene azelate-co-1,2-ethylene succinate), poly(1,4-butylene sebacate-co-1,2-ethylene succinate), poly(1,4-butylene adipate-co-1,4-butylene terephthalate), poly(1,4-butylene sebacate-co-1,4-butylene terephthalate), poly(1,4-butylene azelate-co-1,4-butylene terephthalate), poly(1,4-butylene brassylate-co-1,4-butylene terephthalate), poly(1,4-butylene succinate-co-1,4-butylene terephthalate), poly(1,2-ethylene adipate-co-1,4-butylene terephthalate), poly(1,2-ethylene sebacate-co-1,4-butylene terephthalate), poly(1,2-ethylene azelate-co-1,4-butylene terephthalate), poly(1,2-ethylene brassylate-co-1,4-butylene terephthalate), poly(1,2-ethylene succinate-co-1,4-butylene terephthalate), poly(1,4-butylene adipate-co-1,2-ethylene terephthalate), poly(1,4-butylene sebacate-co-1,2-ethylene terephthalate), poly(1,4-butylene azelate-co-1,2-ethylene terephthalate), poly(1,4-butylene brassylate-co-1,2-ethylene terephthalate), poly(1,4-butylene succinate-co-1,2-ethylene terephthalate), poly(1,4-butylene adipate-co-1,4-butylene 2,5-furandicarboxylate), poly(1,4-butylene sebacate-co-1,4-butylene 2.5- furandicarboxylate), poly(1,4-butylene azelate-co-1,4-butylene 2,5-furandicarboxylate), poly(1,4-butylene brassylate-co-1,4-butylene 2,5-furandicarboxylate), poly(1,4-butylene succinate-co-1,4-butylene 2,5-furandicarboxylate), poly(1,2-ethylene adipate-co-1,4-butylene 2,5-furandicarboxylate), poly(1,2-ethylene sebacate-co-1,4-butylene 2,5-furandicarboxylate), poly(1,2-ethylene azelate-co-1,4-butylene 2,5-furandicarboxylate), poly(1,2-ethylene brassylate-co-1,4-butylene 2,5-furandicarboxylate), poly(1,2-ethylene succinate-co-1,4-butylene 2,5-furandicarboxylate), poly(1,4-butylene adipate-co-1,2-ethylene 2,5-furandicarboxylate), poly(1,4-butylene sebacate-co-1,2-ethylene 2,5-furandicarboxylate), poly(1,4-butylene azelate-co-1,2-ethylene 2,5-furandicarboxylate), poly(1,4-butylene brassylate-co-1,2-ethylene 2,5-furandicarboxylate), poly(1,4-butylene succinate-co-1,2-ethylene 2,5-furandicarboxylate), poly(1,4-butylene terephthalate), poly(1,4-butylene 2,5-furandicarboxylate), poly(1,2-ethylene terephthalate-co-1,4-butylene terephthalate), poly(1,3-propylene terephthalate-co-1,4-butylene terephthalate), poly(1,4-butylene terephthalate-co-1,4-butylene 2,5-furandicarboxylate), poly(1,2-ethylene terephthalate-co-1,4-butylene 2,5-furandicarboxylate), poly(1,3-propylene terephthalate-co-1,4-butylene 2,5-furandicarboxylate), poly(1,2-ethylene terephthalate-co-1,4-butylene 2,5-furandicarboxylate), poly(1,4-butylene terephthalate-co-1,2-ethylene 2,5-furandicarboxylate), their block or random copolymers.

19. Combined process according to any one of claims 1 to 17, in which said polyesters comprising 1,4-butylene dicarboxylate units derive from mixtures comprising at least two saturated aliphatic diacids in turn comprising with respect to the total content of aliphatic diacids at least 50% by moles of one or more saturated aliphatic diacids selected from the group comprising succinic acid, adipic acid, azelaic acid, sebacic acid, brassylic acid, their C1-C24 , preferably C1-C4, esters and mixtures thereof.

## Patentansprüche

1. Kombiniertes Verfahren zur Herstellung von Tetrahydrofuran und Polyestern, die 1,4-Butylen-Dicarboxylateinheiten umfassen, umfassend die Schritte:
1. Verestern und/oder Umestern einer Mischung, die 1,4-Butandiol und zumindest eine Dicarbonsäure, einen Ester, ein Salz oder ein Derivat hiervon umfasst, um ein Oligomer und eine Dampfphase, die Tetrahydrofuran und 1,4-Butandiol umfasst, herzustellen;
2. Abtrennen der Dampfphase, die Tetrahydrofuran und 1,4-Butandiol umfasst, von dem Oligomer;
3. Destillieren der Dampfphase in einer Destillationssäule, die zumindest einen Bodenbereich und zumindest einen gepackten Bereich umfasst;
4. Trennen einer leichten Fraktion, die Tetrahydrofuran und weniger als 1 Gew.-% 1,4-Butandiol umfasst, von einer schweren Fraktion, die 1,4-Butandiol umfasst;
5. Polykondensieren des aus Schritt 1 erhaltenen Oligomers;
worin zumindest einige der Komponenten der Mischung von Schritt 1 vorgemischt werden, bevor sie zu dem Reaktor zugeführt werden.

2. Kombiniertes Verfahren gemäß Anspruch 1, worin die Komponenten bei einer Temperatur niedriger als 70°C vorgemischt werden.

3. Kombiniertes Verfahren gemäß irgendeinem der Ansprüche 1-2, worin der Veresterungs/Umesterungs-Katalysator direkt zu Schritt 1 zugeführt wird.

4. Kombiniertes Verfahren gemäß irgendeinem der Ansprüche 1-2, worin der Veresterungs/Umesterungs-Katalysator zunächst in einem Aliquot von einer oder mehreren Dicarbonsäuren, ihren Estern oder Salzen, und/oder Diolen dispergiert wird, um eine homogene Dispersion in dem Reaktionsmedium zu fördern.

5. Kombiniertes Verfahren gemäß irgendeinem der Ansprüche 1 bis 4, worin der Schritt 1 in zwei oder mehr Veresterungsreaktoren durchgeführt wird, die seriell angeordnet sind.

6. Kombiniertes Verfahren gemäß Anspruch 5, worin der Schritt 1 in zwei Veresterungsreaktoren, die seriell vorgesehen sind, durchgeführt wird, worin der erste Reaktor bei einer Temperatur von 190-230°C und einem Druck von 0,7-1,5 bar mit einer Verweilzeit von 3-7 Stunden betrieben wird, und der zweite Reaktor bei einer Temperatur von 205-250°C und einem Druck von 0,7-1,5 bar mit einer Verweilzeit von 1-3,5 Stunden betrieben wird.

7. Kombiniertes Verfahren gemäß irgendeinem der Ansprüche 1-6, worin der Polykondensationsschritt 5 in der Gegenwart eines Katalysators durchgeführt wird.

8. Kombiniertes Verfahren gemäß Anspruch 7, worin der Katalysator der gleiche ist wie der in Schritt 1 Eingesetzte.

9. Kombiniertes Verfahren gemäß Anspruch 7, worin der im Schritt 1 verwendete Katalysator und das aus der Veresterung- und/oder Umesterungsreaktion resultierende Oligomer zu dem Polykondensationsschritt 5 des Verfahrens zugeführt werden.

10. Kombiniertes Verfahren gemäß irgendeinem der Ansprüche 1 bis 9, worin im Schritt 3 der Teil der Destillationssäule, die mit zumindest einem Boden versehen ist, 15-35 % der Säule ausmacht und der mit einer Packung versehene Teil 85-65 % der Destillationssäule ausmacht.

11. Kombiniertes Verfahren gemäß irgendeinem der Ansprüche 1 bis 10, worin im Schritt 3 die Destillationssäule bei einem Druck zwischen 500 mbar und 1.200 mbar mit Sumpftemperaturen zwischen 150°C und 195°C, Kopftemperaturen zwischen 80°C und 98°C und unter Einsatz eines Rückflussverhältnisses zwischen 0,5 und 2 betrieben wird.

12. Kombiniertes Verfahren gemäß irgendeinem der Ansprüche 1 bis 11, worin ein Oligomerprodukt mit einem Mn < 5.000, gemessen unter Verwendung von Gelpermeationschromatographie (GPC) wie in der Beschreibung angegeben, einer inhärenten Viskosität von 0,03-0,15 dl/g, gemessen unter Verwendung eines Ubbelhode-Viskometers für Lösungen in CHCl₃ bei einer Konzentration von 0,2 g/dl bei 25°C, und einem Endsäuregehalt von 400 mäq/kg oder niedriger am Ende des Schritts 1 erhalten wird.

13. Kombiniertes Verfahren gemäß irgendeinem der Ansprüche 1 bis 12, worin der Schritt 5 bei einer Temperatur von 220-260°C und einem Druck von 350 mbar oder niedriger durchgeführt wird.

14. Kombiniertes Verfahren gemäß irgendeinem der Ansprüche 1 bis 13, worin der Schritt 5 in der Gegenwart einer Gesamtmenge an Katalysator, bezogen auf Titan und Zirkonium, durchgeführt wird, die 80-500 ppm Metall enthält, bezogen auf die Menge an Polyester, die theoretisch durch Reaktion der gesamten zu dem Verfahren zugeführten Dicarbonsäure erhalten werden kann.

15. Kombiniertes Verfahren gemäß irgendeinem der Ansprüche 1 bis 14, umfassend eine oder mehrere Kettenverlängerungs- und/oder reaktive Extrusionsschritte nach Schritt 5.

16. Kombiniertes Verfahren gemäß irgendeinem der Ansprüche 1 bis 15, worin die 1,4-Butylen-Dicarboxylateinheiten der Polyester aus Mischungen von aromatischen Di-Säuren und aliphatischen Di-Säuren stammen, die bezogen auf den Gesamtgehalt von Di-Säuren umfassen:
- von 35 bis 100 mol-%, bevorzugt 40-95 %, von einer oder mehreren aromatischen Di-Säuren oder aromatischen heterocyclischen Dicarbonsäuren, deren Estern oder Salzen;
- von 0 bis 65 mol-%, bevorzugt 5-60 %, von einer oder mehreren aliphatischen Di-Säuren, ihren Estern oder Salzen.

17. Kombiniertes Verfahren gemäß irgendeinem der Ansprüche 1 bis 16, worin die Polyester, die 1,4-Butylen-Dicarboxylateinheiten umfassen, aus Mischungen stammen, die zumindest zwei aromatische Di-Säuren umfassen, die wiederum in Bezug auf den Gesamtgehalt von aromatischen Di-Säuren umfassen:
- von 1 bis 99 mol-%, bevorzugt von 5 bis 95 % oder stärker bevorzugt von 10 bis 80 %, an Terephthalsäure, deren Estern oder deren Salzen;
- von 99 bis 1 mol-%, bevorzugt von 95 bis 5 % und stärker bevorzugt von 90 bis 20 %, an 2,5-Furandicarbonsäure, deren Estern oder Salzen.

18. Kombiniertes Verfahren gemäß irgendeinem der Ansprüche 1 bis 17, worin die Polyester, die 1,4-Butylen-Dicarboxylateinheiten umfassen, ausgewählt sind aus der Gruppe, bestehend aus Poly(1,4-butylensuccinat), Poly(1,4-butylenadipat), Poly(1,4-butylenazelat), Poly(1,4-butylensebacat), Poly(1,4-butylenadipat-co-1,4-butylensuccinat), Poly(1,4-butylenazelat-co-1,4-butylensuccinat), Poly(1,4-butylensebacat-co-1,4-butylensuccinat), Poly(1,2-ethylenadipat-co-1,4-butylensuccinat), Poly(1,2-ethylenazelat-co-1,4-butylensuccinat), Poly(1,2-ethylensebacat-co-1,4-butylensuccinat), Poly(1,4-butylenadipat-co-1,2-ethylensuccinat), Poly(1,4-butylenazelat-co-1,2-ethylensuccinat), Poly(1,4-butylensebacat-co-1,2-ethylensuccinat), Poly(1,4-butylenadipat-co-1,4-butylenterephthalat), Poly(1,4-butylensebacat-co-1,4-butylenterephthalat), Poly(1,4-butylenazelat-co-1,4-butylenterephthalat), Poly(1,4-butylenbrassylat-co-1,4-butylenterephthalat), Poly(1,4-butylensuccinat-co-1,4-butylenterephthalat), Poly(1,2-ethylenadipat-co-1,4-butylenterephthalat), Poly(1,2-ethylensebacat-co-1,4-butylenterephthalat), Poly(1,2-ethylenazelat-co-1,4-butylenterephthalat), Poly(1,2-ethylenbrassylat-co-1,4-butylenterephthalat), Poly(1,2-ethylensuccinat-co-1,4-butylenterephthalat), Poly(1,4-butylenadipat-co-1,2-ethylenterephthalat), Poly(1,4-butylensebacat-co-1,2-ethylenterephthalat), Poly(1,4-butylenazelat-co-1,2-ethylenterephthalat), Poly(1,4-butylenbrassylat-co-1,2-ethylenterephthalat), Poly(1,4-butylensuccinat-co-1,2-ethylenterephthalat), Poly(1,4-butylenadipat-co-1,4-butylen-2,5-furandicarboxylat), Poly(1,4-butylensebacat-co-1,4-butylen-2,5-furandicarboxylat), Poly(1,4-butylenazelat-co-1,4-butylen-2,5-furandicarboxylat), Poly(1,4-butylenbrassylat-co-1,4-butylen-2,5-furandicarboxylat), Poly(1,4-butylensuccinat-co-1,4-butylen-2,5-furandicarboxylat), Poly(1,2-ethylenadipat-co-1,4-butylen-2,5-furandicarboxylat), Poly(1,2-ethylensebacat-co-1,4-butylen-2,5-furandicarboxylat), Poly(1,2-ethylenazelat-co-1,4-butylen-2,5-furandicarboxylat), Poly(1,2-ethylenbrassylat-co-1,4-butylen-2,5-furandicarboxylat), Poly(1,2-ethylensuccinat-co-1,4-butylen-2,5-furandicarboxylat), Poly(1,4-butylenadipat-co-1,2-ethylen-2,5-furandicarboxylat), Poly(1,4-butylensebacat-co-1,2-ethylen-2,5-furandicarboxylat), Poly(1,4-butylenazelat-co-1,2-ethylen-2,5-furandicarboxylat), Poly(1,4-butylenbrassylat-co-1,2-ethylen-2,5-furandicarboxylat), Poly(1,4-butylensuccinat-co-1,2-ethylen-2,5-furandicarboxylat), Poly(1,4-butylenterephthalat), Poly(1,4-butylen-2,5-furandicarboxylat), Poly(1,2-ethylenterephthalat-co-1,4-butylenterephthalat), Poly(1,3-propylenterephthalat-co-1,4-butylenterephthalat), Poly(1,4-butylenterephthalat-co-1,4-butylen-2,5-furandicarboxylat), Poly(1,2-ethylenterephthalat-co-1,4-butylen-2,5-furandicarboxylat), Poly(1,3-propylenterephthalat-co-1,4-butylen-2,5-furandicarboxylat), Poly(1,2-ethylenterephthalat-co-1,4-butylen-2,5-furandicarboxylat), Poly(1,4-butylenterephthalat-co-1,2-ethylen-2,5-furandicarboxylat), ihren Block- oder statistischen Copolymeren.

19. Kombiniertes Verfahren gemäß irgendeinem der Ansprüche 1 bis 17, worin die Polyester, die 1,4-Butylen-Dicarboxylateinheiten umfassen, aus Mischungen stammen, die zumindest zwei gesättigte aliphatische Di-Säuren umfassen, die wiederum in Bezug auf die Gesamtmenge von aliphatischen Di-Säuren mindestens 50 mol-% von einer oder mehreren gesättigten aliphatischen Di-Säuren umfassen, ausgewählt aus der Gruppe bestehend aus Bernsteinsäure, Adipinsäure, Azelainsäure, Sebacinsäure, Brassylicsäure, ihren C1-C24-, bevorzugt C1-C4-Estern, und Mischungen hiervon.

## Revendications

1. Procédé combiné pour la production de tétrahydrofurane et de polyesters comprenant des unités dicarboxylate de 1,4-butylène, comprenant les étapes consistant à :
1. soumettre un mélange comprenant du butane-1,4-diol et au moins un acide dicarboxylique, un ester, un sel ou un dérivé de celui-ci à une estérification et/ou une transestérification pour produire un oligomère et une phase vapeur comprenant du tétrahydrofurane et du butane-1,4-diol ;
2. séparer la phase vapeur comprenant du tétrahydrofurane et du butane-1,4-diol de l'oligomère ;
3. distiller la phase vapeur dans une colonne de distillation comprenant au moins une section à plateaux et au moins une section à garnissage ;
4. séparer une fraction légère comprenant du tétrahydrofurane et moins de 1 % en poids de butane-1,4-diol d'une fraction lourde comprenant du butane-1,4-diol ;
5. polycondenser l'oligomère obtenu à partir de l'étape 1 ;
dans lequel au moins certains des composants du mélange de l'étape 1 sont prémélangés, avant d'être acheminés vers le réacteur.

2. Procédé combiné selon la revendication 1, dans lequel lesdits composants sont prémélangés à une température inférieure à 70 °C.

3. Procédé combiné selon l'une quelconque des revendications 1 à 2, dans lequel le catalyseur d'estérification/transestérification est introduit directement dans l'étape 1.

4. Procédé combiné selon l'une quelconque des revendications 1 à 2, dans lequel le catalyseur d'estérification/transestérification est d'abord dispersé dans une aliquote d'un ou plusieurs acides dicarboxyliques, de leurs esters ou sels et/ou de diols pour favoriser une dispersion homogène dans le milieu réactionnel.

5. Procédé combiné selon l'une quelconque des revendications 1 à 4, dans lequel l'étape 1 est mise en œuvre dans deux ou plus de deux réacteurs d'estérification disposés en série.

6. Procédé combiné selon la revendication 5, dans lequel ladite étape 1 est mise en œuvre dans deux réacteurs d'estérification placés en série, le premier réacteur fonctionnant à une température de 190 à 230 °C et une pression de 0,7 à 1,5 bar, avec un temps de séjour de 3 à 7 heures, et le second réacteur fonctionnant à une température de 205 à 250 °C et une pression de 0,7 à 1,5 bar, avec un temps de séjour de 1 à 3,5 heures.

7. Procédé combiné selon l'une quelconque des revendications 1 à 6, dans lequel l'étape de polycondensation 5 est mise en œuvre en présence d'un catalyseur.

8. Procédé combiné selon la revendication 7, dans lequel le catalyseur est le même que celui utilisé à l'étape 1.

9. Procédé combiné selon la revendication 7, dans lequel le catalyseur utilisé à l'étape 1 et l'oligomère résultant de la réaction d'estérification et/ou de transestérification sont introduits dans l'étape de polycondensation 5 du procédé.

10. Procédé combiné selon l'une quelconque des revendications 1 à 9, dans lequel la partie de la colonne de distillation à l'étape 3 pourvue d'au moins un plateau constitue 15 à 35 % de la colonne et la partie pourvue d'un garnissage constitue 85 à 65 % de la colonne de distillation.

11. Procédé combiné selon l'une quelconque des revendications 1 à 10, dans lequel la colonne de distillation à l'étape 3 fonctionne à des pressions comprises entre 500 mbar et 1200 mbar avec des températures de fond comprises entre 150 °C et 195 °C, des températures de tête comprises entre 80 °C et 98 °C et à l'aide d'un taux de reflux compris entre 0,5 et 2.

12. Procédé combiné selon l'une quelconque des revendications 1 à 11, dans lequel un produit oligomère ayant une Mn, telle que mesurée à l'aide de la chromatographie par perméation de gel (CPG) telle que définie dans la description, < 5000, une viscosité inhérente, telle que mesurée à l'aide d'un viscosimètre Ubbelhode pour des solutions dans du CHCl₃ à une concentration de 0,2 g/dl à 25 °C, de 0,03 à 0,15 dl/g et une acidité terminale inférieure ou égale à 400 méq/kg est obtenu à la fin de l'étape 1.

13. Procédé combiné selon l'une quelconque des revendications 1 à 12, dans lequel ladite étape 5 est mise en œuvre à une température de 220 à 260 °C et une pression inférieure ou égale à 350 mbar.

14. Procédé combiné selon l'une quelconque des revendications 1 à 13, dans lequel ladite étape 5 est mise en œuvre en présence d'une quantité totale de catalyseur à base de titane et de zirconium contenant 80 à 500 ppm de métal par rapport à la quantité de polyester qui peut théoriquement être obtenue par conversion de la totalité de l'acide dicarboxylique introduit dans le procédé.

15. Procédé combiné selon l'une quelconque des revendications 1 à 14, comprenant une ou plusieurs étapes d'allongement de chaîne et/ou d'extrusion réactive après l'étape 5.

16. Procédé combiné selon l'une quelconque des revendications 1 à 15, dans lequel lesdites unités dicarboxylate de 1,4-butylène desdits polyesters sont dérivées de mélanges de diacides aromatiques et de diacides aliphatiques comprenant, par rapport à la teneur totale en diacides :
- de 35 à 100 % en mole, de préférence 40 à 95 %, d'un ou plusieurs diacides aromatiques ou composés dicarboxyliques aromatiques hétérocycliques, de leurs esters ou de leurs sels ;
- de 0 à 65 % en mole, de préférence 5 à 60 %, d'un ou plusieurs diacides aliphatiques, de leurs esters ou de leurs sels.

17. Procédé combiné selon l'une quelconque des revendications 1 à 16, dans lequel lesdits polyesters comprenant des unités dicarboxylate de 1,4-butylène sont dérivés de mélanges comprenant au moins deux diacides aromatiques, comprenant à leur tour par rapport à la teneur totale en diacides aromatiques :
- de 1 à 99 % en mole, de préférence de 5 à 95 % et plus préférablement de 10 à 80 %, d'acide téréphtalique, de ses esters ou de ses sels ;
- de 99 à 1 % en mole, de préférence de 95 à 5 % et plus préférablement de 90 à 20 % d'acide furane-2,5-dicarboxylique, de ses esters ou de ses sels.

18. Procédé combiné selon l'une quelconque des revendications 1 à 17, dans lequel lesdits polyesters comprenant des unités dicarboxylate de 1,4-butylène sont choisis dans le groupe constitué par le poly(succinate de 1,4-butylène), le poly(adipate de 1,4-butylène), le poly(azélate de 1,4-butylène), le poly(sébacate de 1,4-butylène), le poly(adipate de 1,4-butylène-co-succinate de 1,4-butylène), le poly(azélate de 1,4-butylène-co-succinate de 1,4-butylène), le poly(sébacate de 1,4-butylène-co-succinate de 1,4-butylène), le poly(adipate de 1,2-éthylène-co-succinate de 1,4-butylène), le poly(azélate de 1,2-éthylène-co-succinate de 1,4-butylène), le poly(sébacate de 1,2-éthylène-co-succinate de 1,4-butylène), le poly(adipate de 1,4-butylène-co-succinate de 1,2-éthylène), le poly(azélate de 1,4-butylène-co-succinate de 1,2-éthylène), le poly(sébacate de 1,4-butylène-co-succinate de 1,2-éthylène), le poly(adipate de 1,4-butylène-co-téréphtalate de 1,4-butylène), le poly(sébacate de 1,4-butylène-co-téréphtalate de 1,4-butylène), le poly(azélate de 1,4-butylène-co-téréphtalate de 1,4-butylène), le poly(brassylate de 1,4-butylène-co-téréphtalate de 1,4-butylène), le poly(succinate de 1,4-butylène-co-téréphtalate de 1,4-butylène), le poly(adipate de 1,2-éthylène-co-téréphtalate de 1,4-butylène), le poly(sébacate de 1,2-éthylène-co-téréphtalate de 1,4-butylène), le poly(azélate de 1,2-éthylène-co-téréphtalate de 1,4-butylène), le poly(brassylate de 1,2-éthylène-co-téréphtalate de 1,4-butylène), le poly(succinate de 1,2-éthylène-co-téréphtalate de 1,4-butylène), le poly(adipate de 1,4-butylène-co-téréphtalate de 1,2-éthylène), le poly(sébacate de 1,4-butylène-co-téréphtalate de 1,2-éthylène), le poly(azélate de 1,4-butylène-co-téréphtalate de 1,2-éthylène), le poly(brassylate de 1,4-butylène-co-téréphtalate de 1,2-éthylène), le poly(succinate de 1,4-butylène-co-téréphtalate de 1,2-éthylène), le poly(adipate de 1,4-butylène-co-furane-2,5-dicarboxylate de 1,4-butylène), le poly(sébacate de 1,4-butylène-co-furane-2,5-dicarboxylate de 1,4-butylène), le poly(azélate de 1,4-butylène-co-furane-2,5-dicarboxylate de 1,4-butylène), le poly(brassylate de 1,4-butylène-co-furane-2,5-dicarboxylate de 1,4-butylène), le poly(succinate de 1,4-butylène-co-furane-2,5-dicarboxylate de 1,4-butylène), le poly(adipate de 1,2-éthylène-co-furane-2,5-dicarboxylate de 1,4-butylène), le poly(sébacate de 1,2-éthylène-co-furane-2,5-dicarboxylate de 1,4-butylène), le poly(azélate de 1,2-éthylène-co-furane-2,5-dicarboxylate de 1,4-butylène), le poly(brassylate de 1,2-éthylène-co-furane-2,5-dicarboxylate de 1,4-butylène), le poly(succinate de 1,2-éthylène-co-furane-2,5-dicarboxylate de 1,4-butylène), le poly(adipate de 1,4-butylène-co-furane-2,5-dicarboxylate de 1,2-éthylène), le poly(sébacate de 1,4-butylène-co-furane-2,5-dicarboxylate de 1,2-éthylène), le poly(azélate de 1,4-butylène-co-furane-2,5-dicarboxylate de 1,2-éthylène), le poly(brassylate de 1,4-butylène-co-furane-2,5-dicarboxylate de 1,2-éthylène), le poly(succinate de 1,4-butylène-co-furane-2,5-dicarboxylate de 1,2-éthylène), le poly(téréphtalate de 1,4-butylène), le poly(furane-2,5-dicarboxylate de 1,4-butylène), le poly(téréphtalate de 1,2-éthylène-co-téréphtalate de 1,4-butylène), le poly(téréphtalate de 1,3-propylène-co-téréphtalate de 1,4-butylène), le poly(téréphtalate de 1,4-butylène-co-furane-2,5-dicarboxylate de 1,4-butylène), le poly(téréphtalate de 1,2-éthylène-co-furane-2,5-dicarboxylate de 1,4-butylène), le poly(téréphtalate de 1,3-propylène-co-furane-2,5-dicarboxylate de 1,4-butylène), le poly(téréphtalate de 1,2-éthylène-co-furane-2,5-dicarboxylate de 1,4-butylène), le poly(téréphtalate de 1,4-butylène-co-furane-2,5-dicarboxylate de 1,2-éthylène), leurs copolymères à blocs et statistiques.

19. Procédé combiné selon l'une quelconque des revendications 1 à 17, dans lequel lesdits polyesters comprenant des unités dicarboxylate de 1,4-butylène sont dérivés de mélanges comprenant au moins deux diacides aliphatiques saturés comprenant à leur tour par rapport à la teneur totale en diacides aliphatiques au moins 50 % en mole d'un ou plusieurs diacides aliphatiques saturés choisis dans le groupe comprenant l'acide succinique, l'acide adipique, l'acide azélaïque, l'acide sébacique, l'acide brassylique, leurs esters en C1-C24, de préférence en C1-C4, et les mélanges de ceux-ci.
